# EUROPÄISCHE PATENTANMELDUNG

(11) **EP 0 900 794 A1**
(43) Veröffentlichungstag der Anmeldung: **10.03.1999**
(21) Anmeldenummer: 98116198.7
(22) Anmeldetag: 27.08.1998
(51) Int. Cl.: C07D 401/12, C08K 5/3435

(54) **Lichtstabilisatoren auf der Grundlage sterisch gehinderter Amine**

(30) Priorität: 04.09.1997 DE 19738615
(71) Anmelder: Clariant GmbH, 65929 Frankfurt am Main (DE)
(72) Erfinder: Stährfeldt, Thomas, Dr., 86356 Neusäss (DE)

(57) **Zusammenfassung**

Die vorliegende Erfindung bezieht sich auf neue Verbindungen der allgemeinen Formel (I) 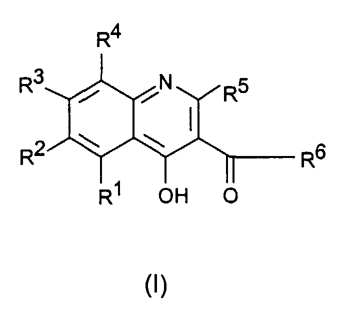 worin die Substituenten die in der Beschreibung definierte Bedeutung haben.

Diese neuen Verbindungen sind ausgezeichnete Stabilisatoren für organisches Material gegen die schädlichen Einflüsse von Licht, Wärme und Sauerstoff.

## Beschreibung

Es ist bekannt, daß organische Materialien durch Licht, Strahlung, Wärme oder Sauerstoff geschädigt werden. Es gibt bereits viele Druckschritten, die Verbindungen zur Stabilisierung von organischem Material gegen diese Einflüsse beschreiben. Es handelt sich dabei meist um Radikalfänger, Hydroperoxidzersetzer, Quencher (Löscher für angeregte Zustände) oder UV-Absorber (vgl. R. Gächter, H. Müller, Plastics Additives Handbook, 3rd Ed., Hanser Verlag, München 1990, S. 133 ff). Im Zusammenhang mit UV-Absorbern handelt es sich in der Regel um Verbindungen auf der Basis von 2-Hydroxybenzophenon, 2-Hydroxyphenylbenzotriazol, Zimtsäureester und Oxanilide (vgl. R. Gächter, H. Müller, Plastics Additives Handbook, 3rd Ed., Hanser Verlag, München 1990, S. 181 ff). Als neuerer UV-Stabilisatortyp kann die Klasse der o-Hydroxy-substituierten Triphenylpyrimidine angesehen werden (DE-A-4416809).

Die genannten Verbindungsklassen weisen oft spezifische Nachteile auf, die neben der erwünschten stabilisierenden Wirkung auftreten. Besonders bezüglich Farbverhalten, Wechselwirkung mit Pigmenten, Verträglichkeit verschiedener, gleichzeitig eingesetzter Stabilisatoren untereinander und mit dem zu stabilisierenden Material, Resistenz gegen Chemikalien und Wasser (Hydrolyseempfindlichkeit), Lagerstabilität, Migrationsverhalten und Verbesserung der Stabilisierung gegen die schädigenden Einflüsse von Wärme und Licht im Langzeitgebrauch besteht ein großer Bedarf nach neuen Stabilisatorklassen.

Derivate der 4-Hydroxychinolin-3-carbonsäure, besonders die freie Säure und die Methyl-/ bzw. Ethylester sind seit einiger Zeit im Zusammenhang mit pharmazeutischen Anwendungen bekannt (vgl. z. B. E. Schroetter et al, Pharmazie (1977), 32(4), 223-5).

Die bisher bekannten Anwendungen beziehen sich auf die Wirkung der 4-Hydroxychinolin-3-carbonsäure und deren Ester gegen Bakterien, Parasiten, Tumore, eitrige Geschwüre, gastrointestinale Fehlfunktionen und als Enzyminhibitoren. Eine UV-stabilisierende Wirkung von Derivaten der 4-Hydroxychinolin-3-carbonsäure ist nicht bekannt.

Substanzen, die einen UV-Stabilisator und ein sterisch gehindertes Amin gleichzeitig im Molekül vereinigen, können besonders wirksame Lichtschutzmittel sein. In diesem Zusammenhang werden z. B. UV-Absorber auf Basis von 2-Hydroxybenzophenon, 2-Hydroxyphenylbenzotriazol und von Zimtsäuren angetroffen. Dies ist u. a. aus EP-A-389427; EP-A-10516; EP-A-16723; EP-A-200190; EP-A-593936; DE-A-4327297 bekannt.

Es wurde nun überraschenderweise gefunden, daß Verbindungen, die ein 4-Hydroxychinolin-3-carbonsäure-Derivat und ein sterisch gehindertes Amin gleichzeitig gemäß der in Formel (I) gezeigten Art im Molekül vereinigen, eine ausgezeichnete Schutzwirkung für organisches Material gegen die schädlichen Einflüsse von Licht, Wärme und Sauerstoff aufweisen. Die Substanzen (I) besitzen im interessanten nahen UV-Bereich in der Regel Extinktionskoeffizienten von 10000 bis 15000 (siehe Experimentalteil).

Gegenstand der vorliegenden Anmeldung sind somit neue Verbindungen der allgemeinen Formel (I)

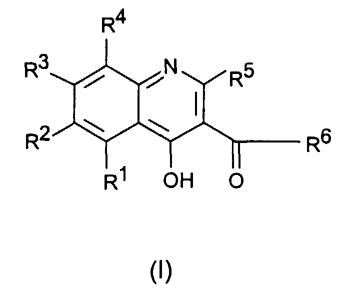

Dabei bedeuten
R¹ bis R⁴ unabhängig voneinander Halogen, H, NO₂, CF₃, CN; C₁-C₂₀-, vorzugsweise C₁-C₁₀-, insbesondere C₁-C₄-Alkyl, -S-Alkyl oder -O-Alkyl; C₆-C₁₄-, vorzugsweise C₆-C₁₀-Aryl, -S-Aryl oder -O-Aryl; C₅-C₁₃-, vorzugsweise C₅-C₁₀-Heteroaryl; C₇-C₂₆-, vorzugsweise C₇-C₁₃-Alkylaryl, -S-Alkylaryl oder -O-Alkylaryl.
   Zwei der Reste R¹ bis R⁴ können zusammen mit dem Grundkörper einen 5-12-gliedrigen, vorzugsweise einen 5-6-gliedrigen, mit Halogen, NO₂, CN, CF₃, C₁-C₂₀-, vorzugsweise C₁-C₁₀-, insbesondere C₁-C₄-Alkyl, -O-Alkyl oder -S-Alkyl, C₆-C₁₄-, vorzugsweise C₆-C₁₀-Aryl, -O-Aryl oder -S-Aryl, C₅-C₁₃-, vorzugsweise C₅-C₁₀-Heteroaryl, C₇-C₂₆-, vorzugsweise C₇-C₁₃-Alkylaryl, -O-Alkylaryl oder -S-Alkylaryl substituierten oder unsubstituierten aliphatischen Ring bilden, der ein oder mehrere Heteroatome beinhalten kann, dieser aliphatische Ring kann insbesonders durch -S-, -O-, -N(H)- unterbrochen sein.
   Von den Resten R¹ bis R⁴ können jeweils zwei benachbarte Reste zusammen mit dem Grundkörper einen weiteren 5-6-gliedrigen, bevorzugt 6-gliedrigen, mit Halogen, NO₂, CF₃, CN, C₁-C₂₀-, vorzugsweise C₁-C₁₀-, insbesondere C₁-C₄-Alkyl, -O-Alkyl oder -S-Alkyl, C₆-C₁₄-, vorzugsweise C₆-C₁₀-Aryl, -O-Aryl oder -S-Aryl, C₅-C₁₃-, vorzugsweise C₅-C₁₀-Heteroaryl, C₇-C₂₆, vorzugsweise C₇-C₁₃-Alkylaryl, -O-Alkylaryl oder -S-Alkylaryl substituierten oder unsubstituierten aromatischen Ring bilden, der ein oder mehrere Heteroatome beinhalten kann oder mit einem weiteren aromatischen Kern annelliert sein kann.
R⁵ bedeutet H, CN oder SR⁷.
R⁷ bedeutet H; mit Halogen, NO₂, CN, CF₃, C₁-C₂₀-, vorzugsweise C₁-C₄-O-Alkyl, C₆-C₁₄-, vorzugsweise C₆-C₁₀-Aryl, O-C₆-C₂₀-, vorzugsweise O-C₆-C₁₀-Aryl oder O-C₇-C₂₆-, vorzugsweise O-C₇-C₁₃-Arylalkyl substituiertes oder unsubstituiertes C₁-C₂₀-Alkyl, vorzugsweise ein unsubstituiertes C₁-C₁₀-Alkyl, insbesonders Methyl; mit Halogen, NO₂, CN, CF₃, O-C₁-C₂₀-, vorzugsweise O-C₁-C₁₀-, insbesondere O-C₁-C₄-Alkyl, O-C₆-C₁₄-, vorzugsweise O-C₆-C₁₀-Aryl, oder O-C₇-C₂₆-, vorzugsweise O-C₇-C₁₃-Arylalkyl substituiertes oder unsubstituiertes C₆-C₁₄-Aryl;
R⁶ bedeutet 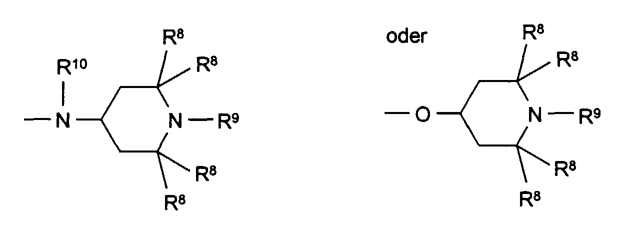
R⁸ bedeutet Wasserstoff oder eine C₁-C₁₂ -Alkylgruppe, vorzugsweise eine C₁-C₄-Alkylgruppe, insbesonders eine Methylgruppe;
R⁹ bedeutet Wasserstoff oder eine C₁-C₂₂ -, vorzugsweise eine C₁-C₅-Alkylgruppe, insbesondere eine Methylgruppe, ein Sauerstoffradikal O*, -OH, -NO, -CH₂CN, Benzyl, Allyl, eine C₁-C₃₀-, vorzugsweise eine C₁-C₁₀-Alkyloxygruppe, eine C₅-C₁₂-, vorzugsweise eine C₅-C₆-Cycloalkyloxygruppe, eine C₆-C₁₀-, vorzugsweise eine C₆-C₇-, insbesonders eine C₆-Aryloxygruppe, wobei der Arylrest zusätzlich noch substituiert sein kann; eine C₇-C₂₀-, vorzugsweise eine C₇-C₁₀-Arylalkyloxygruppe, wobei der Arylrest zusätzlich noch substituiert sein kann, eine C₃-C₁₀-, vorzugsweise eine C₃-C₆-Alkenylgruppe, eine C₃-C₆-Alkinylgruppe, eine C₁-C₁₀-, vorzugsweise eine C₁-C₅-Acylgruppe, Halogen, einen unsubstituierten oder durch C₁-C₄-, vorzugsweise durch C₁-C₂-Alkyl substituierten Phenylrest,
R¹⁰ bedeutet Wasserstoff oder eine C₁-C₁₂ -Alkylgruppe, vorzugsweise eine C₁-C₄-Alkylgruppe, insbesondere eine Butylgruppe.

Die 4-Hydroxychinolin-3-carbonsäureester, also die Vorstufen zu den erfindungsgemäßen Verbindungen (I), können nach dem Stand der Technik hergestellt werden, insbesonders nach der u. a. in Organic Syntheses Coll. Vol. III, S. 274 und in Houben-Weyl, Heterarene II, Teil 1, Band E7a, S. 343 ff beschriebenen Methode durch die thermische Cyclisierung von geeignet substituierten (Arylamino-methylen)-malonsäure-dialkylestern in einem hochsiedenden Lösungsmittel. Diese Vorstufen können auch nach den in DE-A-2431584 beschriebenen Methoden hergestellt werden.
Die cyclisierbaren S-Alkyl-substituierten (Arylamino-methylen)-malonsäuredialkylester für die Synthese von 4-Hydroxychinolin-3-carbonsäureester mit dem -S-Alkyl-Substituent, insbesonders dem -S-CH₃-Substituent, in 2-Position können analog der in Houben-Weyl, Heterarene II, Teil 1, Band E7a, S. 376 publizierten Methode, insbesonders durch Reaktion von Arylthioisocyanat mit dem Natriumsalz der Malonsäure und anschließender Umsetzung mit Alkyliodid hergestellt werden. Die Vorstufe für die Derivate mit dem -CN-Substituent in 2-Position können nach der von S. E. J. Glue, I. T. Kay in Synthesis (1977), 607-8 beschriebenen Methode hergestellt werden.
Die Cyclisierungen dieser Vorstufen zu den erfindungsgemäßen Verbindungen können in einem hochsiedenden organischen Lösungsmittel durchgeführt werden, insbesonders in siedendem o-Dichlorphenol oder in siedendem Diphenylether.

Die Herstellung der erfindungsgemäßen Substanzen ist noch nicht vorbeschrieben. Die neuen Substanzen (I) können durch eine Umesterung hergestellt werden, ausgehend von einem 4-Hydroxychinolin-3-carbonsäureester und einem geeignet substituierten sterisch gehinderten Amin, insbesonders durch Umesterung mit 2,2,6,6-Tetramethylpiperidin-4-ol, 2,2,6,6-Tetramethylpiperidin-4-amin oder N-Butyl-2,2,6,6-tetramethylpiperidin-4-amin. Eine solche Umesterung kann in einem organischen Lösungsmittel, vorzugsweise in einem aromatischen Lösungsmittel, insbesonders in Xylol, durchgeführt werden.

Die Umesterung ist besonders erfolgreich, wenn der Mischung der Edukte noch ein Katalysator zugesetzt wird. Als Katalysator eignen sich z. B. Amide von Metallen der ersten Hauptgruppe des Periodensystems und metallorganische Verbindungen und Oxide von Metallen der vierten Hauptgruppe oder der vierten Nebengruppe des Periodensystems. Geeignete Amide sind Verbindungen der Formel MNH₂ mit M = Li, Na. Metalloxide in diesem Sinne sind z. B. Germanium- oder Zirkondioxid. Geeignete metallorganische Verbindungen von Metallen der vierten Hauptgruppe oder der vierten Nebengruppe des Periodensystems sind z. B. Verbindungen der Formel M'(OR¹¹)₄, worin R¹¹ Alkyl, Phenyl oder Benzyl bedeuten kann und M' das Element Ge, Zr, Sn oder Ti bedeuten kann. Geeignet sind auch Verbindungen der Formel (R¹²)₂SnO, wobei R¹² ein C₄ - C₂₀-Alkyl bedeutet. Als Katalysator besonders geeignet sind Lithiumamid, Dialkylzinnoxide oder Orthotitansäuretetraalkylester, insbesonders Di-n-butyl-zinnoxid, Orthotitansäuretetrabutylester und Orthotitansäuretetraisopropylester.

Die neuen Lichtschutzmittel der Formel (I) können dem zu stabilisierenden organischen Material vor, während oder nach der Polymerisation in fester, geschmolzener, in Lösungsmitteln gelöster Form oder auch als Masterbatch zugegeben werden. Bei dem Zusatz als Feststoff eignen sich die Verbindungen der Formel (I) besonders in fein verteilter Form. Ein Masterbatch eignet sich besonders gut, wenn es den neuen Stabilisator in einer Konzentration von 1 bis 80 %, vorzugsweise aber von 5 - 30 Gew.-% enthält, der Rest im Masterbatch ist ein mit dem zu stabilisierenden Polymer verträgliches Polymer. Besonders eignet sich die Einarbeitung in gelöster Form, wobei die Lösungen den neuen Stabilisator z. B. in 5 - 80 Gew.-%iger Konzentration enthalten können. Sowohl die Lösung, als auch das Masterbatch können zusätzlich noch weitere Stabilisatoren oder Effektstoffe, z. B. weitere UV-Absorber, Lichtschutzmitteln auf Basis von sterisch gehinderten Aminen, Quenchern, Antioxidantien, Pigmente, Säurefänger oder Füllstoffe, enthalten. Die neuen Stabilisatoren werden vorzugsweise so eingesetzt, daß sie im zu stabilisierenden Polymer in einer Konzentration von 0.001 bis 5 Gew.-%, vorzugsweise von 0.02 bis 2 Gew.-%, bezogen auf das organische Material, entweder alleine oder in Kombination mit weiteren Additiven enthalten sind. Unter organischem Material sind beispielsweise Vorprodukte für Kunststoffe, Anstrichmittel, Lacke und Öle, insbesondere jedoch Kunststoffe, Anstrichmittel, Lacke und Öle selbst zu verstehen.

Im besonderen eignen sich die Stabilisatoren der allgemeinen Formel (I) zum Stabilisieren von Folien, Fasern, Bändchen, Multifilamenten, Geweben, Extrusions-, Blasform-, Spritzguß-, Tiefziehartikeln, Pulverlacken, Druckfarben, Tonerfarben, photographischem Material, Pigmenten, Holzbeizen, Leder, Anstrichfarben für Gebäude, Schutzanstrichen für Stahlkonstruktionen, Schmierölen, Maschinenölen, Bitumen, Asphalt, kosmetische oder pharmazeutische Anwendungen, in denen der stabilisierende, insbesonders der UV-absorbierende Charakter der Verbindungen der Formel (I) von Vorteil ist.
Die erfindungsgemäßen Stabilisatoren der Formel (I) können auch in vorteilhafter Weise in Kombinationen mit weiteren Stabilisatoren eingesetzt werden. Das Resultat dieser neuen Kombinationen sind Mischungen mit einem verbesserten Eigenschaftsprofil gegenüber den Einzelkomponenten, wie z. B. synergistische Effekte in der Lichtschutzwirkung.

Gegenstand der vorliegenden Erfindung ist außerdem ein gegen die Einwirkung von Licht, Sauerstoff und Wärme stabilisiertes organisches Material, insbesondere Kunststoffe, Anstrichmittel, Lacke und Öle, welches Verbindungen der Formel (I) in den oben angegebenen Konzentrationen enthält.

Beispiele für derartige Materialien sind in der deutschen Patentanmeldung Nr. 19 719 944.5 auf den Seiten 44 bis 50 beschrieben, worauf hier ausdrücklich Bezug genommen wird.

Das durch die erfindungsgemäßen Verbindungen der Formel (I) oder durch eine geeignete Kombination mit dieser Verbindung stabilisierte organische Material kann gegebenenfalls noch weitere Additive enthalten, beispielsweise Antioxidantien, Lichtstabilisierungsmittel, Metalldesaktivatoren, antistatische Mittel, flammhemmende Mittel, Gleitmittel, Nukleierungsmittel, Säurefänger (basische Costabilisatoren), Pigmente und Füllstoffe. Antioxidantien und Lichtstabilisatoren, die neben den erfindungsgemäßen Verbindungen oder Kombinationen zugesetzt werden, sind beispielsweise Verbindungen auf der Basis sterisch gehinderter Amine oder sterisch gehinderter Phenole oder Schwefel oder Phosphor enthaltende Costabilisatoren.

### Experimenteller Teil

### Allgemeine Vorbemerkungen zur Herstellung der neuen Lichtschutzmittel

Die Vorstufen (4-Hydroxychinolin-3-carbonsäureethylester) für die erfindungsgemäß eingesetzten Verbindungen 1 bis 18 wurden nach dem Stand der Technik hergestellt, insbesonders nach der u. a. in Organic Syntheses Coll. Vol. III, S. 274 und in Houben-Weyl, Heterarene II, Teil 1, Band E7a, S. 343 ff beschriebenen Methoden, d. h. durch die thermische Cyclisierung von geeignet substituierten (Arylamino-methylen)-malonsäure-dialkylestern in einem hochsiedenden Lösungsmittel (Diphenylether, 50 min.). Die cyclisierbaren (Arylamino-methylen)-malonsäure-dialkylester wurden durch Kondensation von primären Aminen mit Ethoxymethylen-malonsäure-dialkylestern hergestellt, wie in Houben-Weyl, Heterarene II, Teil 1, Band E7a, S. 345 beschrieben.

Die Vorstufen für 4-Hydroxychinolin-3-carbonsäureester mit dem -S-CH₃-Substituent in 2-Position wurden analog der in Houben-Weyl, Heterarene II, Teil 1, Band E7a, S. 376 ff publizierten Methode hergestellt, wobei Phenylisothiocyanat mit dem Natriumsalz des Malonsäurediethylesters zu dem entsprechenden (Phenylaminomethylen)-malonsäure-diethylester-thiolat umgesetzt wurde, welches mit Methyliodid zur S-Methylverbindung umgesetzt wurde. Die Cyclisierung dieser Vorstufe zu dem 4-Hydroxychinolin-3-carbonsäureester wurde thermisch in Diphenylether mit etwa 70% Ausbeute durchgeführt. Die Vorstufe für die Derivate mit dem -CN-Substituent in 2-Position wurden nach der von S. E. J. Glue, I.T. Kay in Synthesis (1977), 607-8 beschriebenen Methode hergestellt und in siedendem o-Dichlorbenzol in 23 %iger Ausbeute cyclisiert.

### Beispiele 1 - 18: Herstellung der Verbindungen 1 - 18

0.05 mol eines am aromatischen Grundkörper geeignet substituierten 4-Hydroxychinolin-3-carbonsäureesters werden zusammen mit 0.055 mol eines in 4-Position geeignet substituierten Derivates von 2,2,6,6-Tetramethylpiperidin (4-Hydroxy-2,2,6,6-tetramethylpiperidin, 4-Amino-2,2,6,6-tetramethylpiperidin oder N-Butyl-2,2,6,6-tetramethyl-4-piperidin) und 0.2 g Di-ⁿbutylzinnoxid in 300 ml Xylol bei 136 °C gerührt. Der entstehende Ethanol wird zusammen mit einer kleinen Menge an Xylol über eine Vigreux-Kolonne sukzessive abdestilliert. Wenn das DC das Ende der Umsetzung anzeigt (etwa 48 h), wird der gesamte Ansatz mit 300 ml Wasser intensiv vermischt. Der entstehende Niederschlag wird abgesaugt, das so isolierte Rohprodukt aus DMF umkristallisiert. Ausbeute und Charakterisierung der entsprechenden Verbindungen sind in den Tabellen 1 - 4 dargestellt.

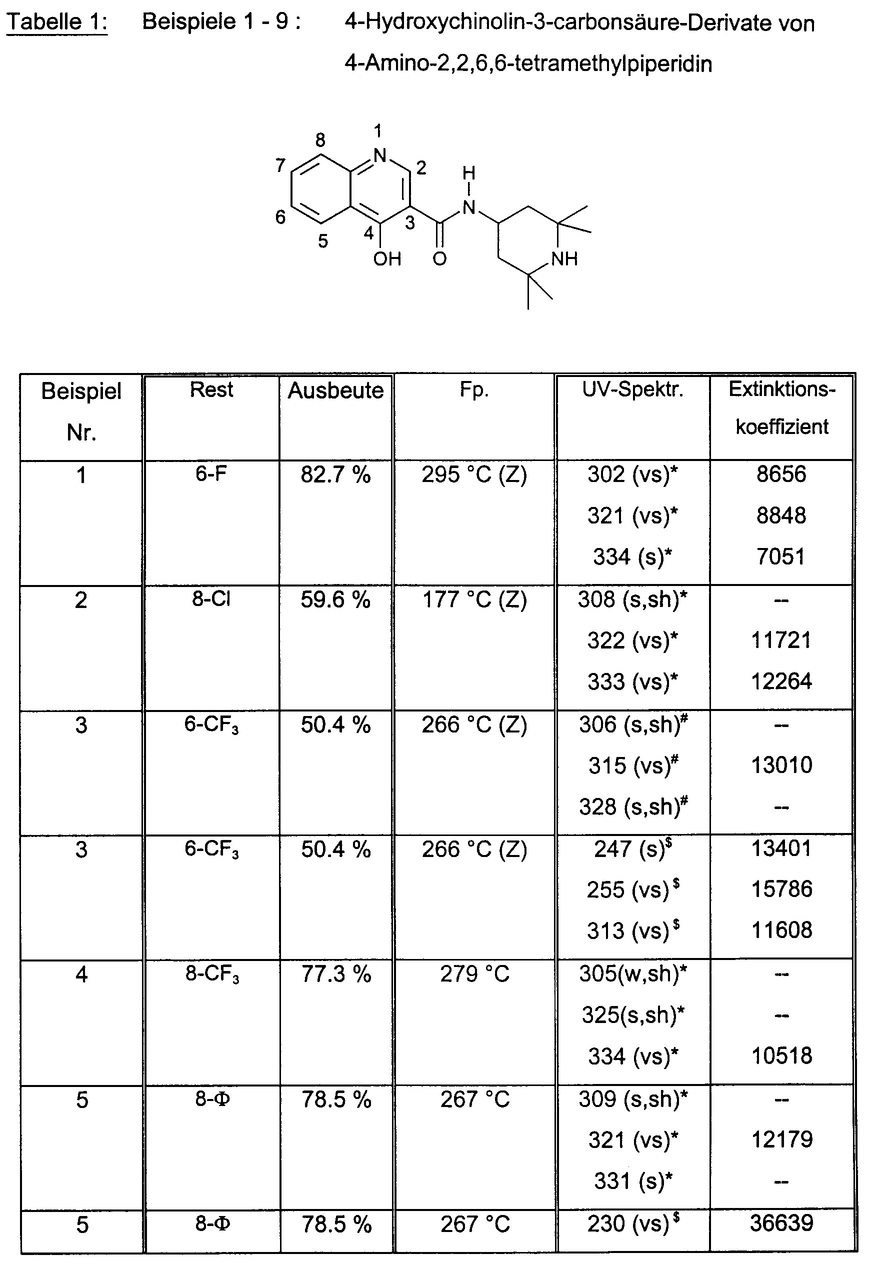

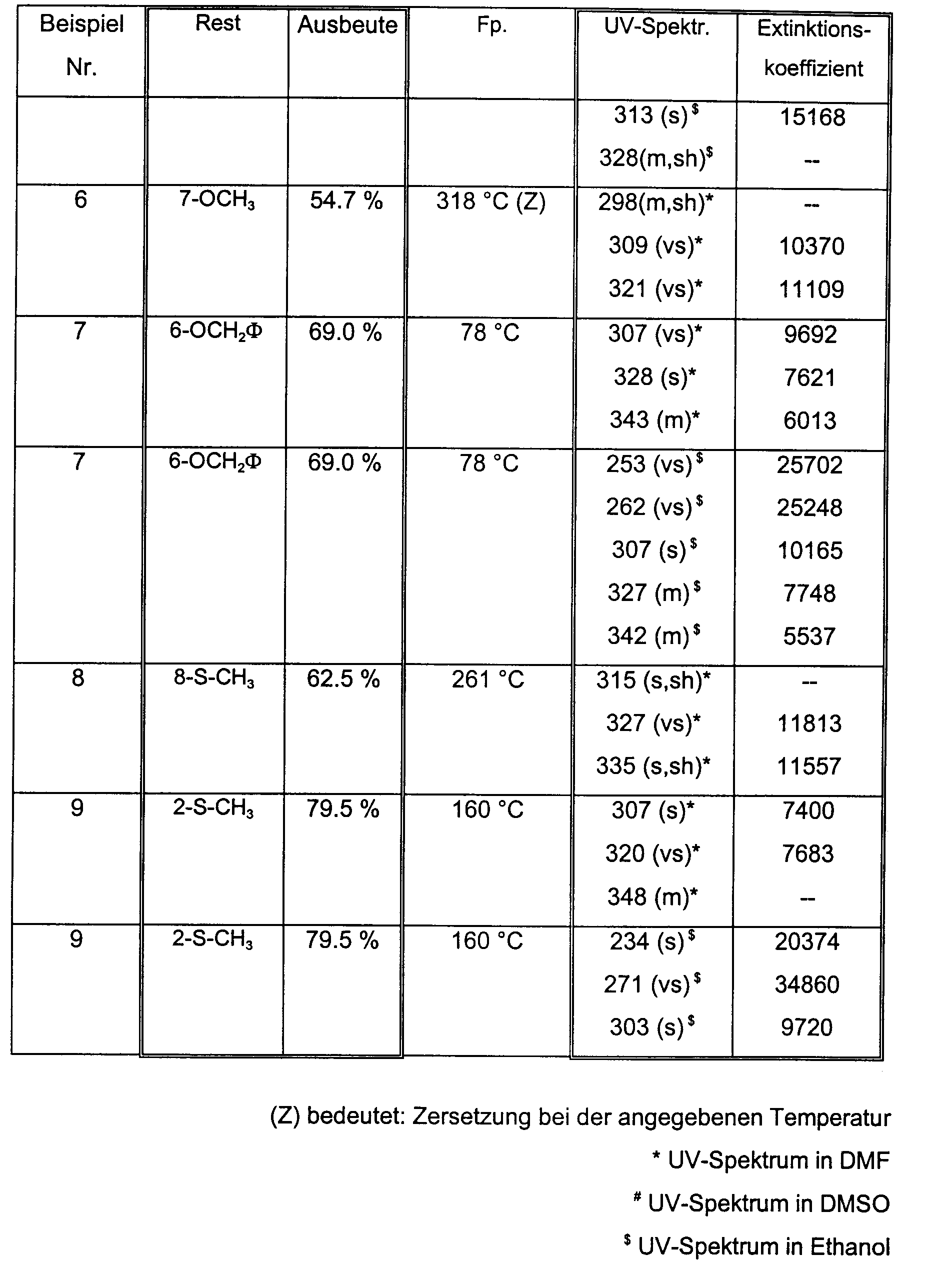

Bei der Bezeichnung der UV-Spektren bedeuten vs = very strong, s=strong, m = medium, w = weak, sh = shoulder.

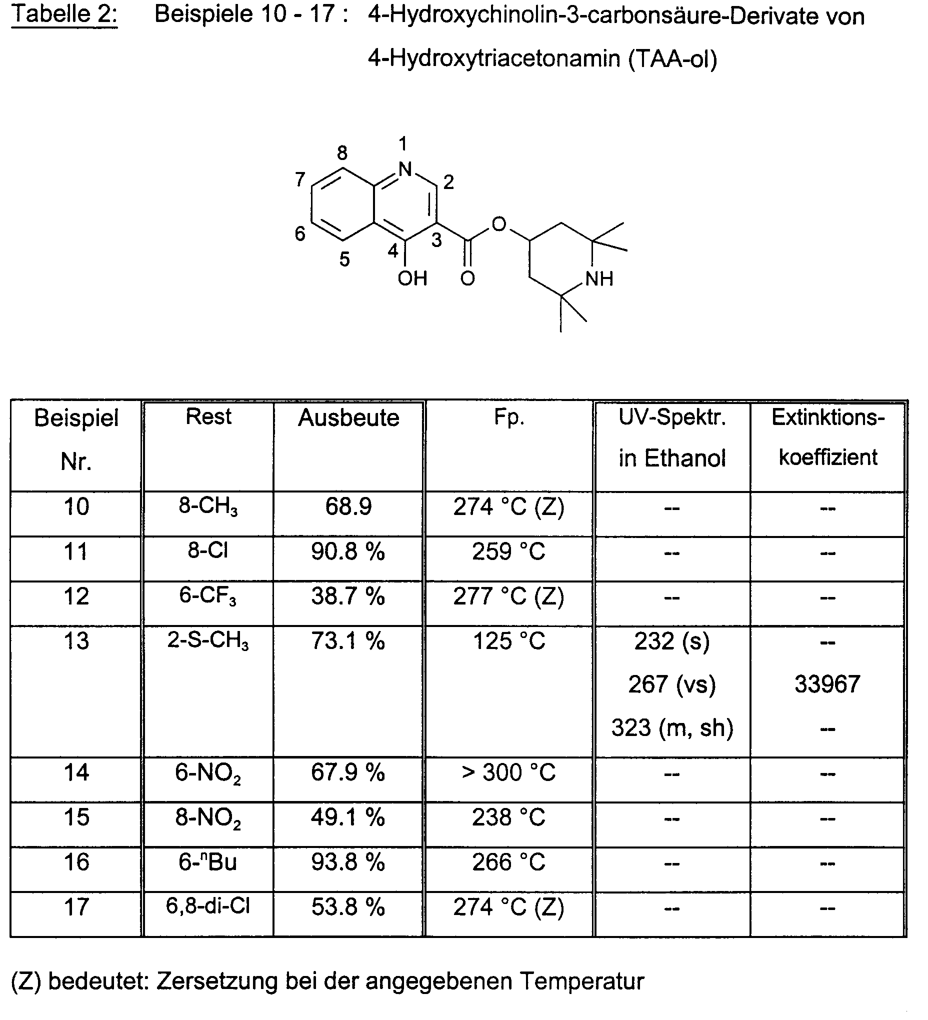

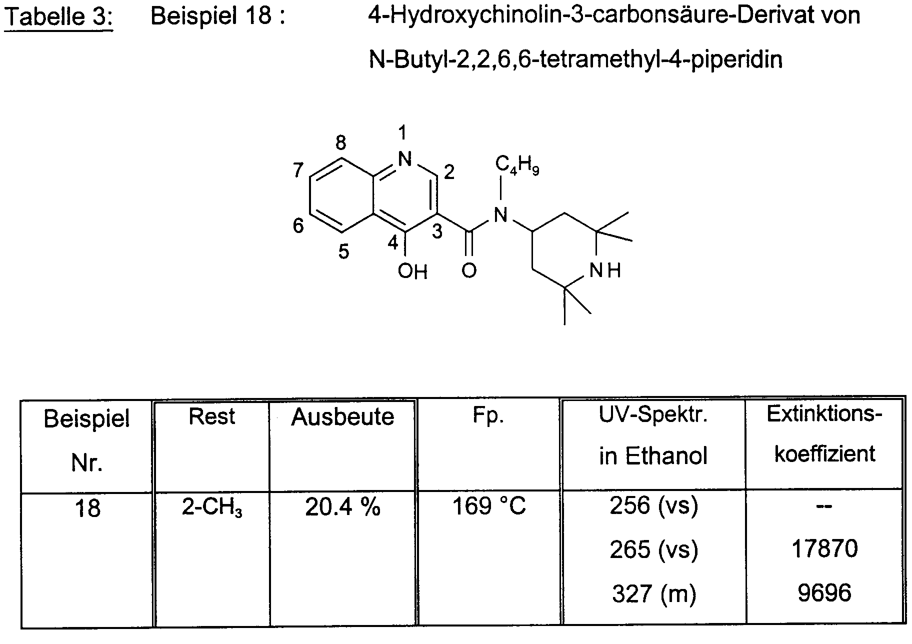

**Tabelle 4**

| Flüchtigkeiten einiger 4-Hydroxychinolin-3-carbonsäure-Derivate | | | | |
|---|---|---|---|---|
| Produkt | **2** | **5** | **6** | **14** |
| Flüchtigkeit | 24 % | 19 % | 18 % | 11 % |

Zur Bestimmung der Flüchtigkeit werden 500 mg der jeweiligen Substanz unter Stickstoff mit einer Geschwindigkeit von 120 °C/h auf 300 °C aufgeheizt und 30 min. bei 300 °C belassen (Pfännchen: Pt, Oberfläche: 3 cm²). In der Tabelle wird der Gewichtsverlust nach 30 min./300 °C angegeben.

### Beispiel 19 - 28: Lichtstabilisierende Wirkung in Polypropylenfolien

100 Gewichtsteile unstabilisiertes Polypropylen (®Hostalen PPK) wurden zusammen mit 0.2 Gewichtsteilen Calciumstearat (Fa. Greven), 0.1 Gewichtsteilen Tris(2,4-di-tert.-butylphenyl)phosphit (®Hostanox PAR 24) und 0.2 Gewichtsteilen des Stabilisators 2 in einem Brabender-Mischer 5 min bei 200°C und 20 U/min geknetet. Aus dieser Mischung wurde bei 190°C eine 200 µm starke Folie gepreßt und die auf diese Weise erhaltenen Prüfkörper in einem Schnellbewitterungsgerät (®Xenotest 1200) belichtet. Als Kriterium der Stabilität der Folie wurde die Veränderung des Carbonylindexes herangezogen. Der Carbonylindex CO wurde hierbei gemäß der Formel CO = E₁₇₂₀/E₂₀₂₀ bestimmt (E = Extinktion). Zu Vergleichszwecken wurde eine Folie unter den gleichen Bedingungen, jedoch ohne den Zusatz eines erfindungsgemäßen Stabilisators getestet. Die Versuchsergebnisse sind in Tabelle 5 zusammengestellt:

**Tabelle 5**

| Veränderung des Carbonylindexes erfindungsgemäß stabilisierter Folien | | |
|---|---|---|
| Beispiel Nr. | Stabilisator | Zeit, nach welcher die Steigerung des Carbonylindexes um 1 Einheit erfolgt (in Stunden) |
| | kein Stabilisator | 190 h |
| 19 | Stabilisator **1** | 380 h |
| 20 | Stabilisator **2** | 300 h |
| 21 | Stabilisator **3** | 310 h |
| 22 | Stabilisator **4** | 330 h |
| 23 | Stabilisator **5** | 370 h |
| 24 | Stabilisator **8** | 270 h |
| 25 | Stabilisator **9** | 300 h |
| 26 | Stabilisator **13** | 370 h |

Tabelle 5 unterstreicht die Lichtschutzwirkung der erfindungsgemäßen Stabilisatoren.

## Patentansprüche

1. Neue Verbindungen der allgemeinen Formel (I), 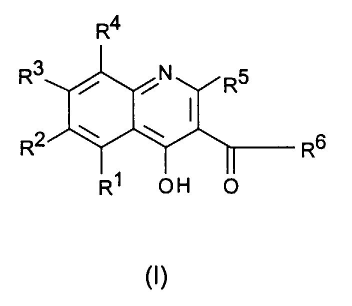 worin
R¹ bis R⁴ unabhängig voneinander Halogen, H, NO₂, CF₃, CN; C₁-C₂₀-Alkyl, S-C₁-C₂₀-Alkyl, O-C₁-C₂₀-Alkyl, C₆-C₁₄-Aryl, S-C₆-C₁₄-Aryl, O-C₆-C₁₄-Aryl, C₅-C₁₃-Heteroaryl, C₇-C₂₆-Alkylaryl, S-C₇-C₂₆-Alkylaryl oder O-C₇-C₂₆-Alkylaryl bedeuten, und wobei
zwei der Reste R¹ bis R⁴ zusammen mit dem Grundkörper einen 5-12-gliedrigen mit Halogen, NO₂, CN, CF₃, C₁-C₂₀-Alkyl, O-C₁-C₂₀-Alkyl, S-C₁-C₂₀-Alkyl, C₆-C₁₄-Aryl, S-C₆-C₁₄-Aryl, O-C₆-C₁₄-Aryl, C₅-C₁₃-Heteroaryl, C₇-C₂₆-Alkylaryl, S-C₇-C₂₆-Alkylaryl oder O-C₇-C-₂₆-Alkylaryl substituierten oder unsubstituierten aliphatischen Ring bilden können, der ein oder mehrere Heteroatome beinhalten kann,
und worin
von den Resten R¹ bis R⁴ jeweils zwei benachbarte Reste zusammen mit dem Grundkörper einen weiteren 5 oder 6-gliedrigen, mit Halogen, NO₂, CN, CF₃, C₁-C₂₀-Alkyl, O-C₁-C₂₀-Alkyl, S-C₁-C₂₀-Alkyl, C₆-C₁₄-Aryl, S-C₆-C₁₄-Aryl, O-C₆-C₁₄-Aryl, C₅-C₁₃-Heteroaryl, C₇-C₂₆-Alkylaryl, S-C₇-C₂₆-Alkylaryl oder O-C₇-C₂₆-Alkylaryl substituierten oder unsubstituierten aromatischen Ring bilden können, der ein oder mehrere Heteroatome beinhalten kann oder mit einem weiteren aromatischen Kern annelliert sein kann,
R⁵ H, CN oder SR⁷ bedeutet,
R⁷ H; mit Halogen, NO₂, CN, CF₃, C₁-C₂₀-O-Alkyl, C₆-C₁₄-Aryl, O-C₆-C₂₀-Aryl oder O-C₇-C₂₆-Arylalkyl substituiertes oder unsubstituiertes C₁-C₂₀-Alkyl, mit Halogen, NO₂, CN, CF₃, O-C₁-C₂₀-Alkyl, O-C₆-C₁₄-Aryl oder O-C₇-C₂₆-Arylalkyl substituiertes oder unsubstituiertes C₆-C₁₄-Aryl bedeutet,
R⁶ einen der folgenden Reste bedeutet, 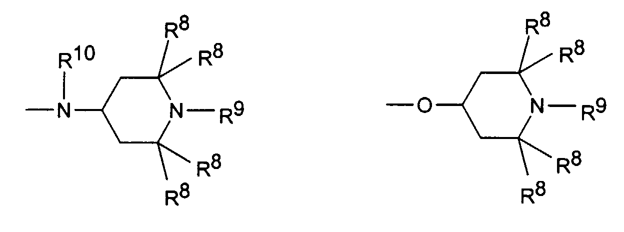
R⁸ Wasserstoff oder eine C₁-C₁₂ -Alkylgruppe bedeutet,
R⁹ Wasserstoff oder eine C₁-C₂₂ -Alkylgruppe, ein Sauerstoffradikal O*, -OH, -NO, -CH₂CN, Benzyl, Allyl, eine C₁-C₃₀-Alkyloxygruppe, eine C₅-C₁₂-Cycloalkyloxygruppe, eine C₆-C₁₀-Aryloxygruppe, wobei der Arylrest zusätzlich noch substituiert sein kann; eine C₇-C₂₀-Arylalkyloxygruppe, wobei der Arylrest zusätzlich noch substituiert sein kann, eine C₃-C₁₀-Alkenylgruppe, eine C₃-C₆-Alkinylgruppe, eine C₁-C₁₀-Acylgruppe, Halogen, einen unsubstituierten oder durch C₁-C₄-Alkyl substituierten Phenylrest, bedeutet,
R¹⁰ Wasserstoff oder eine C₁-C₁₂ -Alkylgruppe bedeutet.

2. Verbindungen gemäß Anspruch 1, dadurch gekennzeichnet, daß R¹ bis R⁴ unabhängig voneinander Halogen, H, NO₂, CF₃, CN, C₁-C₄-Alkyl, S-C₁-C₄-Alkyl, O-C₁-C₄-Alkyl, C₆-C₁₀-Aryl, S-C₆-C₁₀-Aryl, O-C₆-C₁₀-Aryl, C₅-C₁₀-Heteroaryl, C₇-C₁₃-Alkylaryl, S-C₇-C₁₃-Alkylaryl oder O-C₇-C₁₃-Alkylaryl bedeuten, wobei
zwei der Reste R¹ bis R⁴ zusammen mit dem Grundkörper einen 5-6-gliedrigen, mit Halogen, NO₂, CN, CF₃, C₁-C₄-Alkyl, O-C₁-C₄-Alkyl, S-C₁-C₄-Alkyl, C₆-C₁₀-Aryl, S-C₆-C₁₀-Aryl, O-C₆-C₁₀-Aryl, C₅-C₁₀-Heteroaryl, C₇-C₁₃-Alkylaryl, S-C₇-C₁₃-Alkylaryl, oder O-C₇-C₁₃-Alkylaryl substituierten oder unsubstituierten aliphatischen Ring bilden können, der durch -S-, -O-, -N(H)-unterbrochen sein kann,
und wobei
von den Resten R¹ bis R⁴ jeweils zwei benachbarte Reste zusammen mit dem Grundkörper einen weiteren 5 oder 6-gliedrigen, mit Halogen, NO₂, CN, CF₃, C₁-C₄-Alkyl, O-C₁-C₄-Alkyl, S-C₁-C₄-Alkyl, C₆-C₁₀-Aryl, S-C₆-C₁₀-Aryl, O-C₆-C₁₀-Aryl, C₅-C₁₃-Heteroaryl, C₇-C₁₃-Alkylaryl, S-C₇-C₁₃-Alkylaryl oder O-C₇-C₁₃-Alkylaryl substituierten oder unsubstituierten aromatischen Ring bilden können, der ein oder mehrere Heteroatome beinhalten kann oder mit einem weiteren aromatischen Kern annelliert sein kann,
R⁷ H; ein unsubstituiertes C₁-C₁₀-Alkyl; mit Halogen, NO₂, CN, CF₃, O-C₁-C₄-Alkyl, O-C₆-C₁₀-Aryl oder O-C₇-C₁₃-Arylalkyl substituiertes oder unsubstituiertes C₆-C₁₄-Aryl bedeutet,
R⁸ Wasserstoff oder eine C₁-C₄-Alkylgruppe bedeutet,
R⁹ Wasserstoff oder eine C₁-C₅-Alkylgruppe, ein Sauerstoffradikal O*, -OH, -NO, -CH₂CN, Benzyl, Allyl, eine C₁-C₁₀-Alkyloxygruppe, eine C₅-C₆-Cycloalkyloxygruppe, eine C₆-C₇-Aryloxygruppe, wobei der Arylrest zusätzlich noch substituiert sein kann; eine C₇-C₁₀-Arylalkyloxygruppe, wobei der Arylrest zusätzlich noch substituiert sein kann, eine C₃-C₆-Alkenylgruppe, eine C₃-C₆-Alkinylgruppe, eine C₁-C₅-Acylgruppe, Halogen, einen unsubstituierten oder durch C₁-C₂-Alkyl substituierten Phenylrest bedeutet,
R¹⁰ Wasserstoff oder eine C₁-C₄-Alkylgruppe bedeutet.

3. Verbindungen gemäß Anspruch 1, dadurch gekennzeichnet, daß
R¹ bis R⁴ unabhängig voneinander H, Halogen, CF₃, NO₂, C₁-C₄-Alkyl, S-C₁-C₄-Alkyl, O-C₁-C₄-Alkyl, C₆-C₁₀-Aryl, C₇-C₁₃-Alkylaryl, O-C₇-C₁₃-Alkylaryl, S-C₇-C₁₃-Alkylaryl,
R⁵ H oder SR⁷,
R⁷ H, C₁-C₁₀-Alkyl, C₆-C₁₄-Aryl,
R⁸ H oder Methyl,
R⁹ H, Methyl oder eine C₆-Aryloxygruppe, und
R¹⁰ H oder eine Butylgruppe bedeutet.

4. Verwendung der Verbindungen der Formel (I) gemäß den Ansprüchen 1 bis 3 als Stabilisator für organisches Material gegen die schädlichen Einflüsse von Licht, Sauerstoff und Wärme.

5. Verwendung gemäß Anspruch 4, dadurch gekannzeichnet, daß es sich bei dem organischen Material um Kunststoffe, Anstrichmittel, Öle, Lacke oder Vorprodukte davon handelt.

6. Verwendung gemäß Anspruch 4, dadurch gekennzeichnet, daß es sich bei dem organischen Material um pharmazeutische oder kosmetische Produkte handelt.

7. Verwendung der Verbindungen der Formel (I) gemäß den Ansprüchen 1 bis 3 in UV-Filtern, wobei diese Verbindungen in polymerem Material eingebettet oder gelöst sind.

8. Verfahren zum Stabilisieren von organischem Material gegen den durch Licht, Strahlung, Wärme und Sauerstoff verursachten Abbau, dadurch gekennzeichnet, daß man dem zu stabilisierenden Material eine Verbindung der Formel (I) nach den Ansprüchen 1 bis 3 in einer Konzentration von 0.001 - 5 Gew.% zusetzt.

9. Verfahren gemäß Anspruch 8, dadurch gekennzeichnet, daß man 0,1 bis 2,0 Gew.-% zusetzt.

10. Stabilisiertes organisches Material, welches mindestens eine der Verbindungen der allgemeinen Formel (I) gemäß den Ansprüchen 1 bis 3 enthält.

11. Stabilisiertes organisches Material gemäß Anspruch 10, dadurch gekennzeichnet, daß als zusätzliche weitere Additive Antioxidantien, Lichtstabilisatoren, Metalldesaktivatoren, Antistatika, flammhemmende Mittel, Pigmente, Säurefänger oder Füllstoffe zugesetzt werden.

12. Stabilisiertes organisches Material gemäß Anspruch 10 oder 11, dadurch gekennzeichnet, daß es sich um ein Polymeres handelt.
